# EUROPÄISCHE PATENTSCHRIFT

(11) **EP 2 626 018 B1**
(45) Veröffentlichungstag und Bekanntmachung des Hinweises auf die Patenterteilung: **20.03.2019**
(21) Anmeldenummer: 13000129.0
(22) Anmeldetag: 11.01.2013
(51) Int. Cl.: A61B 17/29, A61B 17/00, A61B 90/00

(54) **Medizinisches Instrument**
Medical instrument
Instrument médical

(30) Priorität: 11.02.2012 DE 102012002770
(43) Veröffentlichungstag der Anmeldung: 14.08.2013
(73) Patentinhaber: Karl Storz SE & Co. KG, 78532 Tuttlingen (DE)
(72) Erfinder: Reinauer, Josef, 72488 Sigmaringen (DE); Peschke, Paul, 78589 Dürbheim (DE)
(74) Vertreter: Hofmeister, Frank

(56) Entgegenhaltungen:
- EP-A1- 0 571 057
- EP-A1- 2 269 522
- WO-A1-96/24303
- DE-A1- 19 731 453
- DE-A1- 19 948 031

## Beschreibung

Die Erfindung betrifft ein medizinisches Instrument mit einen Schaft, an dessen distalem Ende ein mindestens ein bewegbares Maulteil aufweisendes Werkzeug angeordnet ist und an dessen proximalem Ende eine mit mindestens einem bewegbaren Griffteil versehene Handhabe angeordnet ist, wobei das bewegbare Maulteil sowie das bewegbare Griffteil über ein Zug- / Druckelement miteinander in Wirkverbindung stehen und wobei zwischen dem bewegbaren Griffteil und dem bewegbaren Maulteil mindestens eine reversibel wirkende Kraftbegrenzungsvorrichtung zum Begrenzen der Kraftübertragung von dem bewegbaren Griffteil auf die mit dem bewegbaren Griffteil in Wirkverbindung stehenden Komponenten angeordnet ist.

Bei derartigen medizinischen Instrumenten kann es sich beispielsweise um Fass-, Halte- und Präparierzangen, Scheren oder dergleichen Instrumente handeln, bei denen durch Handkraft über ein verstellbares Griffteil der Handhabe ein verstellbares Werkzeug, beispielsweise ein verschwenkbares Maulteil, bewegt wird.

Diese bekannten medizinischen Instrumente, die beispielsweise in der endoskopischen Chirurgie Verwendung finden, weisen einen Schaft auf, an dessen distalem Ende ein mindestens ein bewegbares Maulteil aufweisendes Werkzeug angeordnet ist und an dessen proximalem Ende eine mit mindestens einem bewegbaren Griffteil versehene Handhabe angeordnet ist. Zum Öffnen und Schließen des bewegbaren Maulteils sind das bewegbare Maulteil und das bewegbare Griffteil über ein Zug- / Druckelement miteinander verbunden. Die Griffteile der Handhaben sind in der Regel geometrisch so ausgebildet, dass die über die Hand des Benutzers auf das Griffteil ausgeübte Kraft bei der Übertragung auf das mit dem Maulteil gekoppelte Zug- / Druckelement deutlich verstärkt wird, beispielsweise im Verhältnis 10:1, um auch ohne große Kraftanstrengung eine ausreichend hohe Schließkraft am Maulteil zu erzielen.

Um zu verhindern, dass über die Handhabe so große Kräfte auf die Maulteile und / oder das Zug- / Druckelement ausgeübt werden, die zu einer Beschädigung oder gar Zerstörung einzelner Bauteile führen können, ist es aus der Praxis bekannt, medizinische Instrumente mit einer Kraftbegrenzungsvorrichtung zu versehen, die bei Überschreitung einer Grenzlast die miteinander gekoppelten Komponenten reversibel trennt.

Hierzu ist es beispielsweise bekannt, den Kraftübertragungsmechanismus mit einer Sollbruchstelle auszustatten. Diese Art der Kraftbegrenzung gewährleistet zwar einen sicheren Schutz des Instruments, beinhaltet aber den Nachteil, dass das Instrument nicht sofort wieder einsatzfähig ist, da es zur Reparatur der Sollbruchstelle demontiert werden muss.

Ein derartiges medizinisches Instrument mit einer reversibel arbeitenden Kraftbegrenzungsvorrichtung ist beispielsweise aus der DE 197 31 453 C2 bekannt. Bei diesem bekannten medizinischen Instrument ist die Kraftbegrenzung als elastisch verformbarer Teil des Zug- / Druckelements ausgebildet, der sich bei Überschreiten der Grenzlast reversibel verformt.

Weiterhin ist aus der EP 2 269 522 A1 ein gattungsgemäßes medizinisches Instrument bekannt, dessen Kraftbegrenzungsvorrichtung so ausgebildet ist, dass die Kraftbegrenzungsvorrichtung die über die Handhabe aufgebrachte Kraft im Auslösefall im Wesentlichen auf null herabsetzt. Dieses bekannte Instrument hat sich in der Praxis durchaus bewährt, jedoch ist für einzelne Anwendungsfälle vorteilhaft, die über die Handhabe aufgebrachte Kraft auch nach Überschreitung der Grenzlast auf der Handhabe zu belassen.

Das Dokument EP 0 571 057 A1 offenbart einen Nadelhalter mit einen Schaft, an dessen distalem Ende ein mindestens ein bewegbares Maulteil aufweisendes Werkzeug angeordnet ist und an dessen proximalem Ende eine mit mindestens einem bewegbaren Griffteil versehene Handhabe angeordnet ist, wobei das bewegbare Maulteil sowie das bewegbare Griffteil über ein Zug-/Druckelement miteinander in Wirkverbindung stehen und wobei zwischen dem bewegbaren Griffteil und dem bewegbaren Maulteil eine Kraftbegrenzungsvorrichtung zum Begrenzen der bei der Kraftübertragung übertragenen Kraft von dem bewegbaren Griffteil auf die mit dem bewegbaren Griffteil in Wirkverbindung stehenden Komponenten angeordnet ist.

Das Dokument WO 96/24303 A1 offenbart ein medizinisches Instrument mit einen Schaft, an dessen distalem Ende ein hakenförmiges Werkzeug angeordnet ist und an dessen proximalem Ende eine mit mindestens einem bewegbaren Griffteil versehene Handhabe angeordnet ist, wobei das hakenförmige Werkzeug sowie das bewegbare Griffteil über ein Zug-/Druckelement miteinander in Wirkverbindung stehen und wobei zwischen dem bewegbaren Griffteil und dem Werkzeug eine Kraftbegrenzungsvorrichtung zum Begrenzen der bei der Kraftübertragung übertragenen Kraft von dem bewegbaren Griffteil auf die mit dem bewegbaren Griffteil in Wirkverbindung stehenden Komponenten angeordnet ist.

Davon ausgehend liegt der Erfindung die **Aufgabe** zugrunde, ein medizinisches Instrument mit einer Kraftbegrenzungsvorrichtung zu schaffen, die bei Erreichen der Grenzlast ein Beibehalten der aufgebrachten Widerstandskraft gewährleistet.

Die **Lösung** dieser Aufgabenstellung ist erfindungsgemäß durch die Gesamtheit der Merkmale des Anspruchs 1 gekennzeichnet, insbesondere umfasst die Kraftbegrenzungsvorrichtung ein Gehäuse, in dem zwei in Längsrichtung des Gehäuses gegenläufig zueinander verschiebbare Bauteile so angeordnet sind, dass beide Bauteile gegen die Kraft mindestens eines im Gehäuse angeordneten Federelements axial verschiebbar im Gehäuse angeordnet sind, wobei beide Bauteile über mindestens ein auf beide Bauteile einwirkendes und an Anlageflächen der beiden Bauteile anliegendes Kraftübertragungselement miteinander gekoppelt sind und, dass die Kraftbegrenzungsvorrichtung die über das verstellbare Griffteil der Handhabe aufgebrachte Zugkraft (Fz) im Auslösefall im Wesentlichen konstant hält.

Die erfindungsgemäße Ausbildung der Kraftbegrenzungsvorrichtung mit den zwei in Längsrichtung des Gehäuses gegenläufig zueinander verschiebbaren Bauteilen hat den Vorteil, dass die federbelasteten axial verschiebbaren Bauteile ab dem Erreichen einer Grenzlast durch das mindestens eine Kraftübertragungselement mit zunehmender Widerstandskraft kontinuierlich gegeneinander verschoben werden, so dass die über das verstellbare Griffteil der Handhabe ausgeübte Kraft die Grenzlast nicht überschreitet und die Widerstandskraft konstant gehalten wird.

Gemäß einer ersten praktischen Ausführungsform der Erfindung wird vorgeschlagen, dass das mindestens eine Kraftübertragungselement, über das die Zugkraft des verstellbaren Griffteils der Handhabe auf die beiden Bauteile übertragbar ist, als Wälzkörper in Form einer Kugel ausgebildet ist. Diese Art des Kraftübertragungselements stellt eine besonders einfach herzustellende und zu montierende Variante dar. Alternativ dazu kann es sich bei dem Wälzkörper auch um Rollen, Nadeln, Tonnen oder Kegel handeln.

Weiterhin wird mit der Erfindung vorgeschlagen, dass die Anlageflächen, an denen das mindestens eine Kraftübertragungselement an den beiden Bauteilen anliegt, als aufeinander zu geneigte Schrägflächen so ausgebildet sind, dass die Grenzlast ab dem Auslösen der Kraftbegrenzungsvorrichtung eine Winkelfunktion der Kraft des Kraftübertragungselements auf die schräge Anlagefläche des ersten Bauteils ist, wobei die Kraft des Kraftübertragungselements auf die schräge Anlagefläche des ersten Bauteils eine Winkelfunktion der über das verstellbare Griffteil der Handhabe auf die schräge Anlagefläche des zweiten Bauteils ausgeübten Zugkraft ist.

Zur Anordnung der Kraftbegrenzungsvorrichtung wird gemäß einer bevorzugten Ausführungsform der Erfindung vorgeschlagen, dass im bewegbaren Griffteil der Handhabe im Bereich der Kraftübertragung auf das Zug- / Druckelement angeordnet ist. Die Anordnung der Kraftbegrenzungsvorrichtung in der Handhabe ist insbesondere bei endoskopischen Instrumenten vorteilhaft, da bei diesen Instrumenten aufgrund der schlanken Ausgestaltung der Schäfte insbesondere im Bereich der Handhabe ausreichend Platz zur Unterbringung derartiger Vorrichtung vorhanden ist.

Zur Ausbildung der Kraftbegrenzungsvorrichtung wird gemäß einer praktischen Ausführungsform der Erfindung vorgeschlagen, dass das eine Bauteil als am verstellbaren Griffteil der Handhabe gelagerter Zuganker ausgebildet ist und das andere Bauteil als den Zuganker im nicht ausgelösten Zustand der Kraftbegrenzungsvorrichtung koaxial umgebende Hülse ausgebildet ist. Das mindestens eine Federelement ist vorteilhafterweise so im Gehäuse der Kraftbegrenzungsvorrichtung angeordnet, dass dieses unmittelbar und unter Vorspannung an der Hülse anliegt. Die an der Hülse anliegende Vorspannung bestimmt die Grenzlast, ab der die federbelasteten, axial verschiebbaren Bauteile bei zunehmender über das verstellbare Griffteil der Handhabe ausgeübten Kraft kontinuierlich gegeneinander verschoben werden.

Die Anlagefläche des Zugankers, an der das mindestens eine Kraftübertragungselement anliegt, ist erfindungsgemäß als sich zu einem Anlenkpunkt des Zugankers am verschwenkbaren Griffteil hin verjüngender Konus mit flachem, vorzugsweise nicht konstantem Konuswinkel ausgebildet. Der flache Konuswinkel bewirkt, dass bei einer Bewegung des Zugankers auf das Kraftübertragungselement zu die über den Zuganker auf das Kraftübertragungselement ausgeübte Kraft eine große radiale Kraftkomponente auf das Kraftübertragungselement ausübt, mit der dieses seinerseits auf die Anlagefläche der Hülse einwirkt.

Die Anlagefläche der Hülse, an der das mindestens eine Kraftübertragungselement anliegt, ist erfindungsgemäß als sich zu einem Anlenkpunkt des Zugankers am verschwenkbaren Griffteil hin trompetenförmig erweiternder Konus ausgebildet. Der sich trompetenförmig erweiternde Konuswinkel der Hülse bewirkt, dass die über das Kraftübertragungselement auf die Anlagefläche ausgeübte vertikale Kraftkomponente mit zunehmendem Verschieben der Hülse in axialer Richtung fort vom Kraftübertragungselement größer wird und somit eine höhere Druckkraft auf das an der Hülse anliegende Federelement ausübt. Der Konuswinkel nimmt im Verlauf in Richtung Griffteil zu und kann anfangs zwischen 10 und 15 ° liegen, um bis ca. 40 ° anzusteigen. Der Konus kann sowohl Abschnitte mit konstantem Winkel als auch Abschnitte mit kontinuierlich zunehmendem Winkel aufweisen.

Zur Anpassung des medizinischen Instruments an unterschiedliche Einsatzzwecke sowie zur Anpassung an unterschiedliche Werkzeugmaulteile ist die das Auslösen der Kraftbegrenzungsvorrichtung bewirkende Grenzlast über die Vorspannung des mindestens einen Federelements einstellbar.

Weiterhin wird mit der Erfindung vorgeschlagen, dass das Auslösen der Kraftbegrenzungsvorrichtung optisch wahrnehmbar ist, um dem Benutzer zweifelsfrei anzuzeigen, dass die zulässige Grenzlast überschritten und die Kraftbegrenzungsvorrichtung ausgelöst wurde.

Zusätzlich wird eine modifizierte Ausführungsform der Erfindung vorgeschlagen, bei der die Kraftbegrenzungsvorrichtung Druckkräfte begrenzt bzw. in einer weiteren modifizierten Ausführungsform sowohl Druck- als auch Zugkräfte begrenzt.

Schließlich wird mit einer alternativen Ausführungsform der Erfindung vorgeschlagen, dass die Kraftbegrenzungsvorrichtung im Bereich des Zug- / Druckelements angeordnet und beispielsweise als Teil des Zug- / Druckelements, ausgebildet ist.

Weitere Merkmale und Vorteile der Erfindung ergeben sich anhand der zugehörigen Zeichnungen, in denen ein Ausführungsbeispiel eines medizinischen Instruments erfindungsgemäßen nur beispielhaft dargestellt ist, ohne die Erfindung auf dieses Ausführungsbeispiel zu beschränken. In den Zeichnungen zeigt:
- Fig. 1: eine schematische Seitenansicht eines erfindungsgemäßen medizinischen Instruments;
- Fig. 2: eine vergrößerte und teilweise geschnittene schematische Darstellung der Handhabe eines medizinischen Instruments gemäß Fig. 1, den nicht ausgelösten Zustand der Kraftbegrenzungsvorrichtung darstellend;
- Fig. 3: eine vergrößerte isolierte Darstellung der Kraftbegrenzungsvorrichtung gemäß Fig. 2;
- Fig. 4: eine Darstellung gemäß Fig. 3, jedoch die Endstellung der Kraftbegrenzungsvorrichtung darstellend und
- Fig. 5: eine ungeschnittene schematische Seitenansicht des verschwenkbaren Griffteils mit ausgelöster Kraftbegrenzungsvorrichtung.

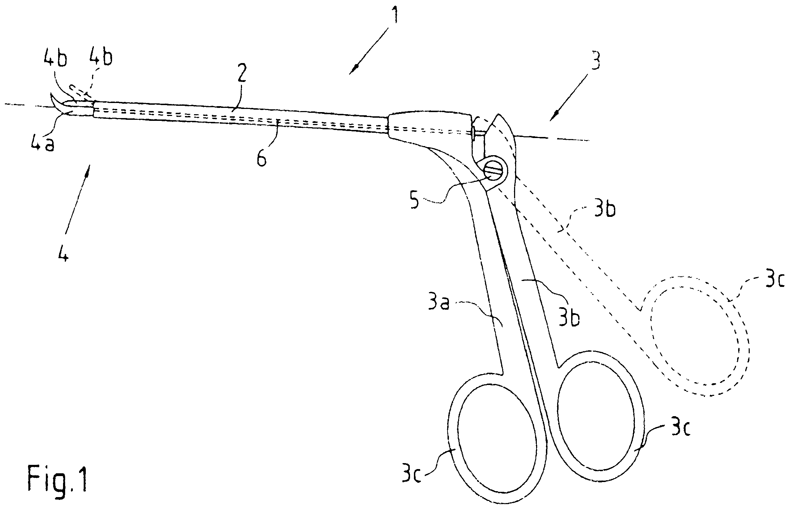

Die Abbildung Fig. 1 zeigt schematisch ein medizinisches Instrument 1, bei dem es sich beispielsweise um eine Fass-, Halte- oder Präparierzange, eine Schere oder dergleichen Instrument handeln kann, bei dem durch Handkraft über ein Werkzeug, beispielsweise ein verschwenkbares Maulteil, bewegt wird.

Das nur schematisch dargestellte medizinische Instrument 1 besteht im Wesentlichen aus einem hohlen Schaft 2, an dessen proximalem Ende eine Handhabe 3 angeordnet ist, die aus einem starren Griffteil 3a und einem gegenüber dem starren Griffteil 3a verschwenkbaren Griffteil 3b besteht. Am distalen Ende des Schaftes 2 ist ein Werkzeug 4 angeordnet, das aus einem starren Maulteil 4a und einem gegenüber dem starren Maulteil 4a verschwenkbaren Maulteil 4b besteht. Welches der beiden Griffteile 3a und 3b der Handhabe 3 verschwenkbar oder anderweitig verstellbar ausgebildet ist, ist für die Funktionsweise des medizinischen Instruments 1 nicht relevant.

Wie weiterhin aus Fig. 1 ersichtlich, stehen das verschwenkbare Maulteil 4b des Werkzeugs 4 und der um eine Schwenkachse 5 verschwenkbare Griffteil 3b der Handhabe 3 über ein in dem hohlen Schaft 2 axial verschiebbar gelagertes Zug- / Druckelement 6 derart in Wirkverbindung miteinander, dass das Verstellen des Griffteils 3b der Handhabe 3 das verschwenkbare Maulteil 4b des Werkzeugs 4 von der geschlossenen Stellung (durchgezogene Darstellung gemäß Fig. 1) in die geöffnete Stellung (gestrichelte Darstellung gemäß Fig. 1) bzw. umgekehrt überführt. Die jeweils zugehörige Stellung des verschwenkbaren Griffteils 3b der Handhabe 3 ist in der Abbildung Fig. 1 ebenfalls durchgezogen (für die geschlossene Stellung) und gestrichelt (für die geöffnete Stellung) dargestellt.

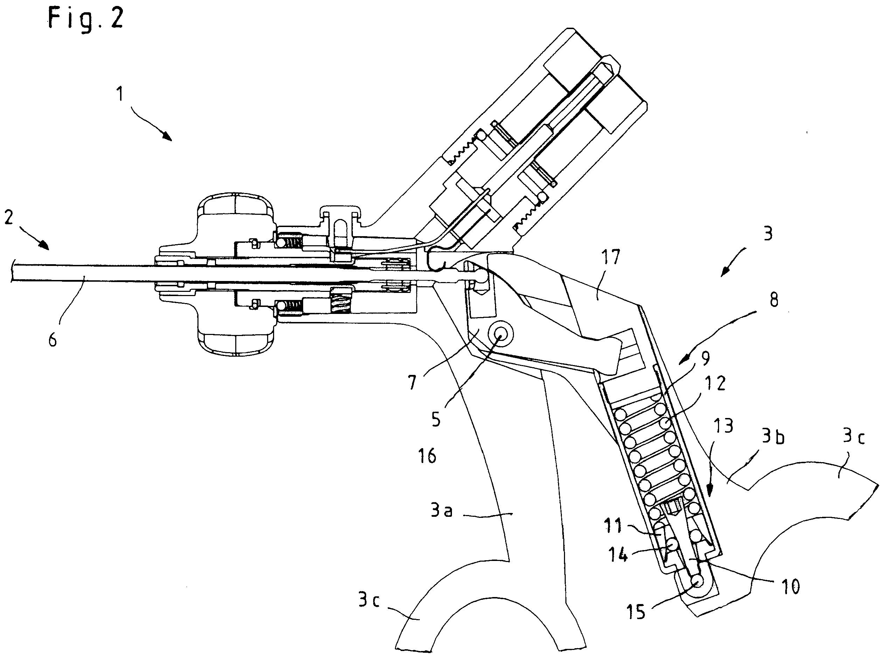

Wie aus einer Zusammenschau der Abbildungen Fig. 1 und 2 ersichtlich, ist das verschwenkbare Griffteil 3b über einen Kipphebel 7 mit dem axial verschiebbaren Zug- / Druckelement 6 verbunden und der Abstand von der Schwenkachse 5 zu der Stelle, an der der Kipphebel 7 mit dem axial verschiebbaren Zug- / Druckelement 6 verbunden ist, wesentlich kürzer als der Abstand von der Schwenkachse 5 zu der am äußeren proximalen Ende des verschwenkbaren Griffteils 3b angeordneten Fingeröse 3c. Das Übersetzungsverhältnis beträgt etwa 10:1, so dass die über die Hand des Benutzers ausgeübte Schließkraft auf das Zehnfache verstärkt wird.

Um zu verhindern, dass über die Handhabe 3 so große Kräfte auf die Maulteile 4a und 4b und/oder das Zug- / Druckelement 6 ausgeübt werden, die zu einer Beschädigung oder gar Zerstörung einzelner Bauteile führen können, weist das medizinische Instrument 1 eine Kraftbegrenzungsvorrichtung 8 auf, die bei Überschreitung einer Grenzlast die übertragene Kraft reversibel begrenzt, so dass sichergestellt ist, dass auch bei zunehmender, auf das verschwenkbare Griffteil 3b ausgeübter Kraft die auf das Werkzeug 4 einwirkende Kraft konstant gehalten wird.

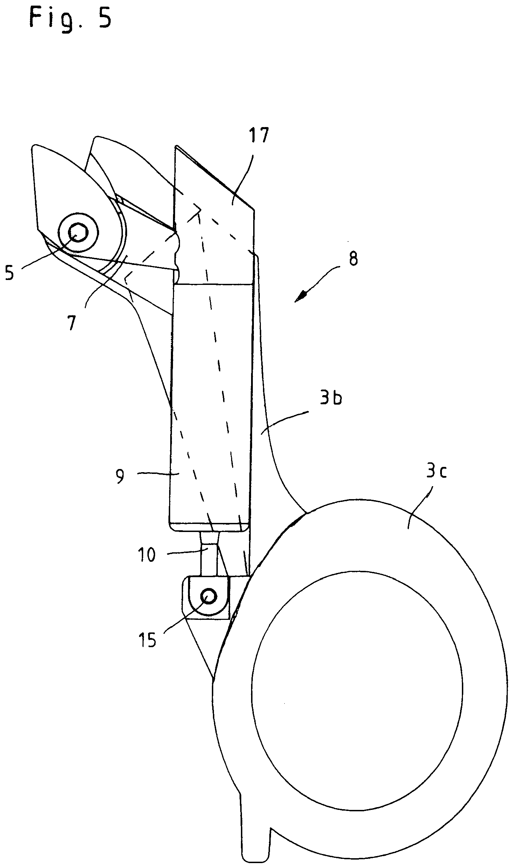

Bei der dargestellten Ausführungsform des medizinischen Instruments 1 ist die Kraftbegrenzungsvorrichtung 8, wie aus Fig. 2 und 5 ersichtlich, im bewegbaren Griffteil 3b der Handhabe 3 im Bereich der Kraftübertragung auf das Zug- / Druckelement 6 angeordnet.

Alternativ zu der dargestellten Anordnung der Kraftbegrenzungsvorrichtung 8 in der Handhabe 3 ist es auch möglich, die Kraftbegrenzungsvorrichtung 8 im Bereich des Zug- / Druckelements 6 anzuordnen und beispielsweise als Teil des Zug- / Druckelements 6 auszubilden.

Der Aufbau und die Arbeitsweise der Kraftbegrenzungsvorrichtung 8 ist den Abbildungen Fig. 2 bis 5 zu entnehmen.

Die Kraftbegrenzungsvorrichtung 8 besteht im Wesentlichen aus einem Gehäuse 9, in dem zwei in Längsrichtung des Gehäuses 9 gegenläufig zueinander verschiebbare Bauteile 10 und 11 so angeordnet sind, dass beide Bauteile 10 und 11 gegen die Kraft mindestens eines im Gehäuse 9 angeordneten Federelements 12 axial verschiebbar im Gehäuse 9 angeordnet sind, wobei beide Bauteile 10 und 11 über mindestens ein auf beide Bauteile 10 und 11 einwirkendes Kraftübertragungselement 13 miteinander gekoppelt sind, das die Zugkraft F_{Z} des verstellbaren Griffteils 3b der Handhabe 3 auf die beiden Bauteile 10 und 11 überträgt.

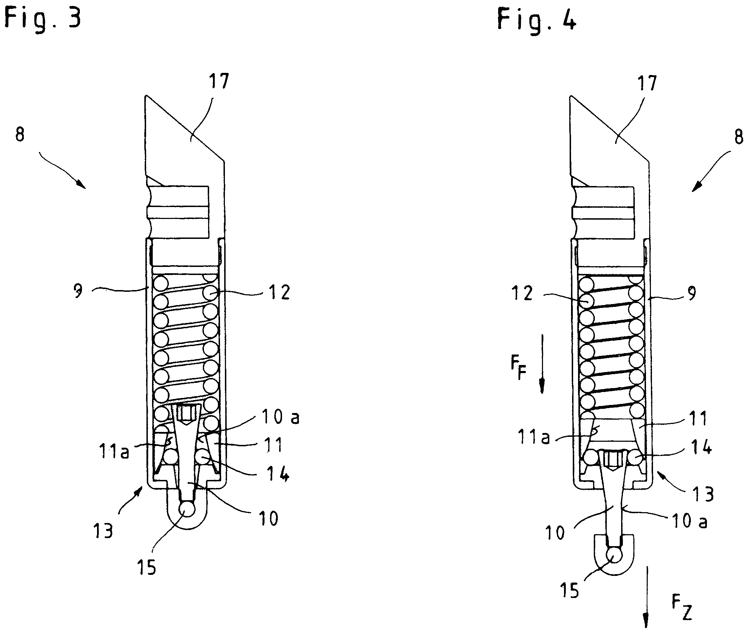

Wie insbesondere aus Fig. 3 und 4 ersichtlich, sind die Anlageflächen 10a und 11a der beiden Bauteile 10 und 11, an denen das Kraftübertragungselement 13 an den beiden Bauteilen 10 und 11 anliegt, als aufeinander zu geneigte Schrägflächen ausgebildet.

Das mindestens eine an den Anlageflächen 10a und 11a anliegende Kraftübertragungselement 13 ist bei der in den Abbildungen Fig. 2 bis 4 dargestellten Ausführungsform als Kugel 14 ausgebildet.

Wie weiterhin aus den Abbildungen Fig. 2 bis 4 ersichtlich, sind die beiden gegenläufig zueinander verschiebbaren, im Gehäuse 9 der Kraftübertragungsvorrichtung 8 gelagerten Bauteile 10 und 11 als am verstellbaren Griffteil 3b der Handhabe 3 gelagerter Zuganker 10 und als den Zuganker 10 im Wesentlichen koaxial umgebende Hülse 11 ausgebildet. Die Übertragung der Zugkraft F_{Z} des verstellbaren Griffteils 3b der Handhabe 3 über das Kraftübertragungselement 13 auf die beiden Bauteile 10 und 11 erfolgt über den Zuganker 10, der an einem Anlenkpunkt 15 am verstellbaren Griffteil 3b der Handhabe 3 gelagert ist. Das Federelement 12 liegt bei dieser Ausführungsform unter Vorspannung auf der dem Zuganker 10 abgewandten Seite direkt an der Hülse 11 an.

Bei der dargestellten Ausführungsform sind als Kraftübertragungselemente 13 sechs Kugeln 14 vorgesehen, die an den entsprechenden Anlageflächen 10a und 11a des Zugankers 10 sowie der Hülse 11 anliegen.

Die Anlagefläche 10a des Zugankers 10, an der die Kugeln 14 anliegen, ist als sich zum Anlenkpunkt 15 des Zugankers 10 am verschwenkbaren Griffteil 3b hin verjüngender Konus mit flachem Konuswinkel ausgebildet. Der flache Konuswinkel bewirkt, dass bei einer Bewegung des Zugankers 10 auf die Kugeln 14 zu die über den Zuganker 10 auf die Kugeln 14 ausgeübte Kraft eine große radiale Kraftkomponente auf die Kugeln 14 ausübt, mit der diese ihrerseits auf die Anlagefläche 11a der Hülse 11 einwirken.

Die Anlagefläche 11a der Hülse 11, an der die Kugeln 14 anliegen, ist als sich zum Anlenkpunkt 15 des Zugankers 10 am verschwenkbaren Griffteil 3b hin trompetenförmig erweiternder Konus ausgebildet. Der trompetenförmig sich erweiternde Konuswinkel der Hülse 11 bewirkt ab dem Erreichen einer Grenzkraft, dass die über die Kugeln 14 auf die Anlagefläche 11a ausgeübte vertikale Kraftkomponente mit zunehmendem Verschieben der Hülse 11 in axialer Richtung fort vom Kraftübertragungselement (nach oben in Fig. 3 und 4) größer wird und somit eine höhere Druckkraft auf das an der Hülse 11 anliegende Federelement 12 ausübt. Die Konuswinkel des Zugankers 10 und der Hülse 11 sind so ausgelegt, dass die Druckkraft auf das an der Hülse 11 anliegende Federelement 12 die gemäß Federkonstante zunehmende Kraft des Federelements 12 kompensiert.

Aufgrund der konischen Ausgestaltung der Anlageflächen 10a und 11a ist die gegen die Federkraft F_{F} des Federelements 12 wirkende Gegenkraft der Kraftbegrenzungsvorrichtung 8 eine Winkelfunktion der Kraft des Kraftübertragungselements 13 auf die schräge Anlagefläche 11a der Hülse 11, wobei die Kraft des Kraftübertragungselements 13 auf die schräge Anlagefläche 11a der Hülse 11 ihrerseits eine Winkelfunktion der über das verstellbare Griffteil 3b der Handhabe 3 auf die schräge Anlagefläche 10a des Zugankers 10 ausgeübten Zugkraft F_{Z} ist.

Die dargestellte Kraftbegrenzungsvorrichtung 8 arbeitet wie folgt:
Beim Verschwenken des verschwenkbaren Griffteils 3b der Handhabe 3 um die Schwenkachse 5 wird der am Anlenkpunkt 15 angelenkte und im Gehäuse 9 gelagerte Zuganker 10 mitbewegt und wirkt infolge der ausgeübten Zugkraft F_{Z}, unter Zwischenschaltung der Kugeln 14 sowie der Hülse 11, gegen die Vorspannung des Federelements 12, während das Gehäuse 9 der Bewegung des Zugankers folgt.

Diese Bewegung bewirkt, dass der am oberen Ende des Gehäuses 9 eingehängte und um die Schwenkachse 5 verschwenkbare Kipphebel 7 verschwenkt wird und über eine formschlüssige Einhängung das Zug- / Druckelement 6 zum Schließen des verschwenkbaren Maulteils 4b des Werkzeugs 4 nach proximal zieht.

Kann nun, beispielsweise durch das Ergreifen eines Gegenstandes mit den Maulteilen 4a und 4b des Werkzeugs 4, das verschwenkbare Maulteil 4b nicht weiter geschlossen werden und somit auch das Zug- / Druckelement 6 nicht weiter nach proximal gezogen werden, so steigt aufgrund der Lagerung des Kipphebels 7 am Gehäuse 9 die Zugkraft F_{Z} am Gehäuse und überschreitet die von der Vorspannung des Federelements 12 ausgeübte Federkraft F_{F}.

Das weitere Herausziehen des Zugankers 10 aus dem Gehäuse 9 der Kraftbegrenzungsvorrichtung 8 bewirkt aufgrund der konischen Anlagefläche 10a des Zugankers 10 eine weiter zunehmende Druckkraft auf die zur Kraftübertragung auf die Hülse 11 dienenden Kugeln 14. Aufgrund des flachen Konuswinkels der Anlagefläche 10a des Zugankers 10 an den Kugeln 14 weist die über den Zuganker 10 auf die Kugeln 14 ausgeübte Druckkraft eine sehr hohe radiale Kraftkomponente auf.

Diese hohe radiale Kraftkomponente, mit der die Kugeln 14 ihrerseits auf die Anlagefläche 11a der Hülse 11 einwirken, bewirkt aufgrund der sich nach unten trompetenförmig erweiternden Anlagefläche 11a der Hülse 11 an den Kugeln 14 ein kontinuierliches Ansteigen der über die Kugeln 14 auf die Hülse 11 ausgeübte vertikale Kraftkomponente und somit zu einem Komprimieren des Federelements 11, während sich die Gesamtlänge der Kraftbegrenzungsvorrichtung 8 vergrößert, d. h. der Abstand vom oberen Ende des Gehäuses 9 bis zum Anlenkpunkt 15 wird länger.

Die Ausbildung der Kraftbegrenzungsvorrichtung 8 mit den zwei in Längsrichtung des Gehäuses 9 gegenläufig zueinander verschiebbaren Bauteilen Zuganker 10 und Hülse 11, die über die an den beiderseitigen konischen Anlageflächen 10a und 11a anliegenden, zur Kraftübertragung dienenden Kugeln 14 in Wirkverbindung miteinander stehen, bewirkt ,dass die federbelasteten axial verschiebbaren Bauteile 10 und 11 bis zum Erreichen eines Endanschlags 16 über die Kugeln 14 mit zunehmender Widerstandskraft kontinuierlich gegeneinander verschoben werden, so dass die über das verstellbare Griffteil 3b der Handhabe 3 ausgeübte Kraft die Grenzlast nicht überschreitet und die Widerstandskraft konstant gehalten wird.

Das Herausziehen des Zugankers 10 nach Überschreiten der Grenzlast bewirkt eine Verlängerung des Verschwenkweges des verschwenkbaren Griffteils 3b bis zu einem Endanschlag 16, so dass die weitere Bewegung des verschwenkbaren Griffteils 3b ohne Auswirkung auf das Werkzeug 4 am distalen Ende bleibt.

Die erhaltene Federspannung des Federelements 12 ist ausreichend, um beim Zurückschwenken des verschwenkbaren Griffteils 3b in die Ausgangsposition gemäß Fig. 2 die Hülse 11 sowie die Kugeln 14 reversibel wieder zurück in die Ausgangsposition zu bringen, so dass die Kraftbegrenzungsvorrichtung 8 erneut einsatzbereit ist.

Bei der in den Abbildungen Fig. 2 bis 5 dargestellten Ausführungsform ist die Kraftbegrenzungsvorrichtung 8 zusätzlich mit einer Kappe 17 ausgestattet, die beim Auslösen der Kraftbegrenzungsvorrichtung 8 aus dem verschwenkbaren Griffteil 3b der Handhabe 3 heraustritt, weil das verschwenkbare Griffteil 3b sich ab dem Erreichen der Grenzlast weiterbewegen lässt, ohne eine weitergehende Kippbewegung des Kipphebels 7 zu verursachen. Dadurch wird dem Benutzer das Überschreiten der Grenzlast und das Auslösen der Kraftbegrenzungsvorrichtung 8 angezeigt, wie dies der Abbildung Fig. 5 zu entnehmen ist.

Die Kraftbegrenzungsvorrichtung 8 gemäß dem beschriebenen Ausführungsbeispiel ist für die Begrenzung von Zugkräften ausgelegt. Um Druckkräfte begrenzen zu können, ist eine Modifikationen dahingehend erforderlich, dass die Länge der Kraftbegrenzungsvorrichtung 8 beim Überschreiten der Grenzkraft abnimmt. Dazu kann der Zuganker 10 durch einen Druckbolzen ersetzt werden, dessen Konizität ausgehend von einem Anlenkpunkt abnimmt sowie den Einsatz einer Hülse mit einer der Hülse 11 entgegengesetzten Konizität. Je nach räumlicher Anordnung der Hülse kann anstelle des drückenden Federelements 12 auch eine Zugfeder Verwendung finden, die sowohl mit dem Gehäuse als auch mit der Hülse verbunden ist. Um sowohl Zug- als auch Druckkräfte begrenzen zu können, muss ein kombinierter Zug- / Druckanker zum Einsatz kommen, welcher mit zwei gegenüberliegenden Hülsen 11 und Federelementen 12 zusammenwirkt.

Ein solchermaßen ausgebildetes medizinisches Instrument 1 zeichnet sich dadurch aus, dass die reversibel arbeitende Kraftbegrenzungsvorrichtung 8 auch nach dem Erreichen der Grenzlast eine bleibende Widerstandskraft beibehält.

**Bezugszeichenliste**

| | | | |
|---|---|---|---|
| 1 | medizinisches Instrument | 11a | Anlagefläche |
| 2 | Schaft | 12 | Federelement |
| 3 | Handhabe | 13 | Kraftübertragungselement |
| 3a | starres Griffteil | 14 | Kugel |
| 3b | verschwenkbares Griffteil | 15 | Anlenkpunkt |
| 3c | Fingeröse | 16 | Endanschlag |
| 4 | Werkzeug | 17 | Kappe |
| 4a | starres Maulteil | | |
| 4b | verschwenkbares Maulteil | | |
| 5 | Schwenkachse | | |
| 6 | Zug- / Druckelement | F_{F} | Federkraft |
| 7 | Kipphebel | F_{Z} | Zugkraft |
| 8 | Kraftbegrenzungsvorrichtung | | |
| 9 | Gehäuse | | |
| 10 | Bauteil / Zuganker | | |
| 10a | Anlagefläche | | |
| 11 | Bauteil / Hülse | | |

## Patentansprüche

1. Medizinisches Instrument mit einen Schaft (2), an dessen distalem Ende ein mindestens ein bewegbares Maulteil (4b) aufweisendes Werkzeug (4) angeordnet ist und an dessen proximalem Ende eine mit mindestens einem bewegbaren Griffteil (3b) versehene Handhabe (3) angeordnet ist, wobei das bewegbare Maulteil (4b) sowie das bewegbare Griffteil (3b) über ein Zug- / Druckelement (6) miteinander in Wirkverbindung stehen und wobei zwischen dem bewegbaren Griffteil (3b) und dem bewegbaren Maulteil (4b) mindestens eine reversibel wirkende Kraftbegrenzungsvorrichtung (8) zum Begrenzen der bei der Kraftübertragung übertragenen Kraft von dem bewegbaren Griffteil (3b) auf die mit dem bewegbaren Griffteil (4b) in Wirkverbindung stehenden Komponenten bewegbares Maulteil (4b) und Zug- / Druckelement (6) angeordnet ist,
**dadurch gekennzeichnet,**
**dass** die Kraftbegrenzungsvorrichtung (8) ein Gehäuse (9) umfasst, in dem zwei in Längsrichtung des Gehäuses (9) gegenläufig zueinander verschiebbare Bauteile (10, 11) so angeordnet sind, dass beide Bauteile (10, 11) gegen die Kraft mindestens eines im Gehäuse (9) angeordneten Federelements (12) axial verschiebbar im Gehäuse (9) angeordnet sind, wobei beide Bauteile (10, 11) über mindestens ein auf beide Bauteile (10, 11) einwirkendes und an Anlageflächen (10a, 11a) der beiden Bauteile (10, 11) anliegendes Kraftübertragungselement (13) miteinander gekoppelt sind und, dass die Kraftbegrenzungsvorrichtung (8) die über das verstellbare Griffteil (3b) der Handhabe (3) aufgebrachte Zugkraft (F_{Z}) im Auslösefall im Wesentlichen konstant hält.

2. Medizinisches Instrument nach Anspruch 1, **dadurch gekennzeichnet, dass** über das mindestens eine Kraftübertragungselement (13) die Zugkraft (F_{Z}) des verstellbaren Griffteils (3b) der Handhabe (3) auf die beiden Bauteile (10, 11) übertragbar ist.

3. Medizinisches Instrument nach Anspruch 1 oder 2, **dadurch gekennzeichnet, dass** das mindestens eine Kraftübertragungselement (13) als Kugel (14) ausgebildet ist.

4. Medizinisches Instrument nach einem der Ansprüche 1 bis 3, **dadurch gekennzeichnet, dass** Anlageflächen (10a, 11a), an denen das mindestens eine Kraftübertragungselement (13) an den beiden Bauteilen (10, 11) anliegt, als aufeinander zu geneigte Schrägflächen ausgebildet sind.

5. Medizinisches Instrument nach einem der Ansprüche 1 bis 4, **dadurch gekennzeichnet, dass** das eine Bauteil (10) als am verstellbaren Griffteil (3b) der Handhabe (3) gelagerter Zuganker (10) ausgebildet ist und das andere Bauteil (11) als den Zuganker (10) im Wesentlichen koaxial umgebende Hülse (11) ausgebildet ist.

6. Medizinisches Instrument nach Anspruch 5, **dadurch gekennzeichnet, dass** das mindestens eine Federelement (12) unmittelbar an der Hülse (11) anliegt.

7. Medizinisches Instrument nach den Anspruch 5 oder 6, **dadurch gekennzeichnet, dass** die Anlagefläche (10a) des Zugankers (10), an der das mindestens eine Kraftübertragungselement (13) anliegt, als sich zu einem Anlenkpunkt (15) des Zugankers (10) am verschwenkbaren Griffteil (3b) hin verjüngender Konus mit flachem Konuswinkel ausgebildet ist.

8. Medizinisches Instrument nach den Anspruch 5 oder 6, **dadurch gekennzeichnet**, die Anlagefläche (11a) der Hülse (11), an der das mindestens eine Kraftübertragungselement (13) anliegt, als sich zu einem Anlenkpunkt (15) des Zugankers (10) am verschwenkbaren Griffteil (3b) hin trompetenförmig erweiternder Konus ausgebildet ist.

9. Medizinisches Instrument nach einem der Ansprüche 5 bis 8, **dadurch gekennzeichnet, dass** die Grenzlast, ab der die Kraftbegrenzungsvorrichtung (8) auslöst, eine Winkelfunktion der Kraft des Kraftübertragungselements (13) auf die schräge Anlagefläche (11a) der Hülse (11) ist, wobei die Kraft des Kraftübertragungselements (13) auf die schräge Anlagefläche (11a) der Hülse (11) eine Winkelfunktion der über das verstellbare Griffteil (3b) der Handhabe (3) auf die schräge Anlagefläche (10a) des Zugankers (10) ausgeübten Zugkraft (F_{Z}) ist.

10. Medizinisches Instrument nach einem der Ansprüche 1 bis 9, **dadurch gekennzeichnet, dass** die Kraftbegrenzungsvorrichtung (8) im bewegbaren Griffteil (3b) der Handhabe (3) im Bereich des Kraftübertragungsmechanismus auf das Zug- / Druckelement (6) angeordnet ist.

11. Medizinisches Instrument nach einem der Ansprüche 1 bis 10, **dadurch gekennzeichnet, dass** die das Auslösen der Kraftbegrenzungsvorrichtung (8) bewirkende Grenzlast über das Federelement (12) einstellbar ist.

12. Medizinisches Instrument nach Anspruch 11, **dadurch gekennzeichnet, dass** die Vorspannung des Federelements (12) der Grenzlast entspricht.

13. Medizinisches Instrument nach einem der Ansprüche 1 bis 12, **dadurch gekennzeichnet, dass** das Auslösen der Kraftbegrenzungsvorrichtung (8) optisch wahrnehmbar ist.

14. Medizinisches Instrument nach einem der Ansprüche 1 bis 4, **dadurch gekennzeichnet, dass** die Kraftbegrenzungsvorrichtung (8) im Bereich des Zug- / Druckelements (6) angeordnet ist.

## Claims

1. A medical instrument comprising a shaft (2), at the distal end of which there is arranged a tool (4) having at least one movable jaw part (4b), and at the proximal end of which there is arranged a handle (3) provided with at least one movable handle part (3b), wherein the movable jaw part (4b) and the movable handle part (3b) are operably linked with one another via a pull/push element (6), and wherein at least one reversibly acting force limiting device (8) is arranged between the movable handle part (3b) and the movable jaw part (4b) for limiting the force transmitted during the force transmission from the movable handle part (3b) to the components movable jaw part (4b) and pull/push element (6) which are operably linked to movable handle part (4b),
**characterized in,**
**that** the force limiting device (8) comprises a housing (9) in which two displaceable components (10, 11) are arranged in longitudinal direction of the housing (9) in opposite direction in such a way that both components (10, 11) are arranged in the housing axially displaceably against the force of at least one spring element (12) arranged in the housing (9), wherein both components (10, 11) are coupled together by at least one force transmission element (13) acting on both components (10, 11) and abutting on abutment surfaces (10a, 11a) of the two components (10, 11), and that the force limiting device (8) keeps the pulling force (F_{z}) applied via the adjustable handle part (3b) of the handle (3) substantially constant when tripped.

2. The medical instrument according to claim 1, **characterized in that** the pulling force (F_{z}) of the adjustable handle part (3b) of the handle (3) is transferable to the two components (10, 11) by means of the at least one force transmission element (13).

3. The medical instrument according to claim 1 or 2, **characterized in that** the at least one force transmission element (13) is configured as a ball (14).

4. The medical instrument according to any one of claims 1 to 3, **characterized in that** abutment surfaces (10a, 11a), on which the at least one force transmission element (13) abuts the two components (10, 11), are configured as inclined surfaces inclined towards one another.

5. The medical instrument according to any one of claims 1 to 4, **characterized in that** the one component (10) is configured as a tie rod (10) mounted on the adjustable handle part (3b) of the handle (3) and that the other component (11) is configured as a sleeve (11) substantially coaxially surrounding the tie rod (10).

6. The medical instrument according to claim 5, **characterized in that** the at least one spring element (12) abuts directly on the sleeve (11).

7. The medical instrument according to claim 5 or 6, **characterized in that** the abutment surface (10a) of the tie rod (10) on which abuts the at least one force transmission element (13) is configured as a cone with a flat cone angle tapering towards a hinging point (15) of the tie rod (10) on the pivotable handle part (3b).

8. The medical instrument according to claim 5 or 6, **characterized in that** the abutment surface (11a) of the sleeve (11) on which abuts the at least one force transmission element (13) is configured as a trumpet-shaped widening cone towards a hinging point (15) of the tie rod (10) on the pivotable handle part (3b).

9. The medical instrument according to any one of claims 5 to 8, **characterized in that** the limit load at which the force limiting device (8) trips is an angular function of the force of the force transmission element (13) on the inclined abutment surface (11a) of the sleeve (11), wherein the force of the force transmission element (13) on the inclined abutment surface (11a) of the sleeve (11) is an angular function of the pulling force (F_{z}) exerted by the adjustable handle part (3b) of the handle (3) on the inclined abutment surface (10a) of the tie rod (10).

10. The medical instrument according to any one of claims 1 to 9, **characterized in that** the force limiting device (8) is arranged in the movable handle part (3b) of the handle (3) in the area of the force transmission mechanism on the pull/push element (6).

11. The medical instrument according to any one of claims 1 to 10, **characterized in that** the limit load causing the tripping of the force limiting device (8) is adjustable via the spring element (12).

12. The medical instrument according to claim 11, **characterized in that** the bias of the spring element (12) corresponds to the limit load.

13. The medical instrument according to any one of claims 1 to 12, **characterized in that** the tripping of the force limiting device (8) is visually perceptible.

14. The medical instrument according to any one of claims 1 to 4, **characterized in that** the force limiting device (8) is arranged in the area of the pull/push element (6).

## Revendications

1. Instrument médical muni d'une tige (2), à l'extrémité distale de laquelle est agencé au moins un outil (4) présentant une pièce de mâchoire mobile (4b) et à l'extrémité proximale de laquelle est agencée une poignée (3) pourvue d'au moins une partie de préhension mobile (3b), dans lequel la pièce de mâchoire mobile (4b) et la partie de préhension mobile (3b) se trouvent en liaison active entre elles au moyen d'un élément de traction/poussée (6), et dans lequel au moins un dispositif limiteur de force (8) à action réversible, destiné à limiter la force transmise de la partie de préhension mobile (3b) aux composants en liaison active avec la partie de préhension mobile (4b) lors du transfert de force d'un élément de traction/poussée (6), est agencé entre la partie de préhension mobile (3b) et la pièce de mâchoire mobile (4b),
**caractérisé**
**en ce que** le dispositif limiteur de force (8) comprend un boîtier (9), dans lequel deux composants (10, 11) déplaçables l'un vers l'autre en sens contraires dans la direction longitudinale du boîtier (9) sont agencés de telle manière que lesdits deux composants (10, 11) sont déplaçables axialement dans le boîtier (9) contre la force d'au moins un élément de ressort (12) agencé dans le boîtier (9), dans lequel lesdits deux composants (10, 11) sont accouplés l'un à l'autre au moyen d'au moins un élément de transmission de force (13) agissant sur les deux composants (10, 11) et appliqué contre des surfaces de butée (10a, 11a) des deux composants (10, 11), et **en ce que** le dispositif limiteur de force (8) maintient sensiblement constante la force de traction (F_{Z}) appliquée sur la partie de préhension mobile (3b) de la poignée (3) en cas de déclenchement.

2. Instrument médical selon la revendication 1, **caractérisé en ce que** la force de traction (F_{Z}) de la partie de préhension mobile (3b) de la poignée (3) est transmissible aux deux composants (10, 11) au moyen dudit au moins un élément de transmission de force (13).

3. Instrument médical selon la revendication 1 ou 2, **caractérisé en ce que** ledit au moins un élément de transmission de force (13) est réalisé en forme de sphère (14).

4. Instrument médical selon l'une des revendications 1 à 3, **caractérisé en ce que** les surfaces de butée (10a, 11a), contre lesquelles est appliqué au moins un élément de transmission de force (13) sur les composants (10, 11) sont réalisées comme des surfaces inclinées l'une vers l'autre.

5. Instrument médical selon l'une des revendications 1 à 4, **caractérisé en ce que** le premier composant (10) est réalisé comme un tirant (10) monté sur la partie de préhension mobile (3b) de la poignée (3), et **en ce que** l'autre composant (11) est réalisé comme une douille (11) entourant sensiblement coaxialement le tirant (10).

6. Instrument médical selon la revendication 5, **caractérisé en ce que** ledit au moins un élément de ressort (12) est appliqué directement contre la douille (11).

7. Instrument médical selon la revendication 5 ou 6, **caractérisé en ce que** la surface de butée (10a) du tirant (10) contre laquelle ledit au moins un élément de transmission de force (13) est appliqué, est réalisée comme un cône effilé vers un point d'articulation (15) du tirant (10) sur la partie de préhension mobile (3b), à angle de cône plat.

8. Instrument médical selon la revendication 5 ou 6, **caractérisé en ce que** la surface de butée (11a) de la douille (11), contre laquelle ledit au moins un élément de transmission de force (13) est appliqué, est réalisée comme un cône s'évasant en forme de trompette vers un point d'articulation (15) du tirant (10) sur la partie de préhension mobile (3b).

9. Instrument médical selon l'une des revendications 5 à 8, **caractérisé en ce que** la charge limite à partir de laquelle le dispositif limiteur de force (8) se déclenche est une fonction trigonométrique de la force de l'élément de transmission de force (13) sur la surface de butée inclinée (11a) de la douille (11), dans lequel la force de l'élément de transmission de force (13) sur la surface de butée inclinée (11a) de la douille (11) est une fonction trigonométrique de la force de traction (F_{Z}) appliquée sur la partie de préhension mobile (3b) de la poignée (3) sur la surface de butée inclinée (10a) du tirant (10).

10. Instrument médical selon l'une des revendications 1 à 9, **caractérisé en ce que** le dispositif limiteur de force (8) est agencé dans la partie de préhension mobile (3b) de la poignée (3) au niveau du mécanisme de transmission de force sur l'élément de traction/poussée (6).

11. Instrument médical selon l'une des revendications 1 à 10, **caractérisé en ce que** la charge limite provoquant le déclenchement du dispositif limiteur de force (8) est réglable au moyen de l'élément de ressort (12).

12. Instrument médical selon la revendication 11, **caractérisé en ce que** la précontrainte de l'élément de ressort (12) correspond à la charge limite.

13. Instrument médical selon l'une des revendications 1 à 12, **caractérisé en ce que** le déclenchement du dispositif limiteur de force (8) est perceptible visuellement.

14. Instrument médical selon l'une des revendications 1 à 4, **caractérisé en ce que** le dispositif limiteur de force (8) est agencé au niveau de l'élément de traction/poussée (6).
